# NOUVEAU FASCICULE DE BREVET EUROPEEN

(11) **EP 0 712 422 B2**
(45) Date de publication et mention de la décision concernant l'opposition: **13.08.2003**
(45) Mention de la délivrance du brevet: 13.01.1999
(21) Numéro de dépôt: 94923770.5
(22) Date de dépôt: 02.08.1994
(51) Int. Cl.: C08J 3/03, A61K 9/127, A61K 7/00

(54) **PROCEDE DE PREPARATION D'UNE COMPOSITION AQUEUSE SOUS FORME DE GEL ET L'UTILISATION DE CE PROCEDE**
VERFAHREN ZUR HERSTELLUNG EINER WÄSSRIGEN GELARTIGEN ZUSAMMENSETZUNG UND DESSEN VERWENDUNG
METHOD FOR PREPARING A GELLED AQUEOUS COMPOSITION AND USE THEREOF

(30) Priorité: 04.08.1993 FR 9309607
(43) Date de publication de la demande: 22.05.1996
(73) Titulaire: LVMH RECHERCHE, 92752 Nanterre (FR)
(72) Inventeur: ILIOPOULOS, Ilias, F-75012 Paris (FR); CARTALAS-SARRAZIN, Anne, F-92130 Issy-les-Moulineaux (FR); LOYEN, Karine, F-76620 Le Havre (FR); AUDEBERT, Roland, F-95320 Saint-Leu-la-Forêt (FR); MEYBECK, Alain, F-92400 Courbevoie (FR); TRANCHANT, Jean-François, F-45760 Marigny-les-Usages (FR)
(74) Mandataire: Giraud, Françoise
(86) Numéro de dépôt international: FR9400971
(87) Numéro de publication internationale: WO95004774

(56) Documents cités:
- EP-A- 0 346 995
- WO-A-87/00848
- WO-A-91/08280
- WO-A-92/20735
- GB-A- 2 166 107
- PROGRESS IN COLLOID & POLYMER SCIENCE vol. 89 , 1992 , DARMSTADT, DE pages 118 - 121 MAGNY B. ET AL 'Interactions between Hydrophobically Modified Polymers and Surfactants' cité dans la demande
- JOURNAL OF THE CHEMICAL SOCIETY, FARADAY TRANSACTIONS vol. 86, no. 12 , 1990 , LONDON pages 2253 - 2261 STREY R. ET AL 'Dilute Lamellar and L3 Phases in the Binary Water-C12E5 System' cité dans la demande

## Description

La présente invention concerne un nouveau procédé de préparation d'une composition aqueuse se présentant sous forme de gel ainsi que l'utilisation de ce procédé pour stabiliser une suspension aqueuse de particules liquides ou solides, en particulier de vésicules, et plus particulièrement de liposomes.

On sait que les polymères hydrosolubles trouvent de nombreuses applications dans des domaines industriels très variés tels que la cosmétologie, la récupération assistée du pétrole, les additifs alimentaires.

Ils sont très souvent utilisés pour contrôler la rhéologie des formulations aqueuses, car avec peu de matière sèche, on peut obtenir des solutions très visqueuses, voire même des gels. Une classe particulière de ces polymères hydrosolubles est celle des polymères hydrosolubles associatifs.

On citera deux ouvrages récents tout particulièrement consacrés à ce type de polymères: Water Soluble Polymers, ACS Symposium Series 467, ed. Shalaby W. Shalaby et al., Am. Chem. Soc. Washington (1991), pp. 82-200 et Polymers in aqueous media, ed. J. Edward Glass, Advances in Chemistry Series n°223, Am. Chem. Soc. Washington DC (1989).

Les polymères associatifs sont des polymères constitués d'une chaîne principale hydrophile et de chaînes latérales hydrophobes en nombre relativement faible par rapport aux motifs hydrophiles de la chaîne principale. Leur comportement en solution résulte de la compétition entre les propriétés hydrophobes et hydrophiles de sa structure. Les motifs hydrophobes ont tendance à former des agrégats constituant des points de réticulation entre les chaînes macromoléculaires.

Sur le plan rhéologique, les polymères hydrosolubles associatifs présentent un pouvoir viscosifiant très élevé dans l'eau, une bonne tenue de leur viscosité en milieu salin et un comportement réversible vis-à-vis de la contrainte (rhéo-fluidifiant).

L'étude de systèmes aqueux contenant des polymères et des tensioactifs présente un grand intérêt compte tenu de leurs nombreuses applications industrielles dans des domaines aussi variés que les peintures, la cosmétique, ou la récupération assistée du pétrole.

Dans ces systèmes, des agrégats mixtes polymère/tensioactif peuvent se former, stablisés par différents types d'interactions : électrostatiques, dipolaires, liaison hydrogène. Les polymères hydrosolubles associatifs peuvent interagir avec les tensioactifs plus spécifiquement grâce à leur parties hydrophobes.

Ainsi, il ressort des publications suivantes: I. Iliopoulos et al., Langmuir 1991, 7, 617 ; B. Magny et al., Prog. Colloid. Polym. Sci. 1992, 89, 118 que l'addition d'un tensioactif cationique, anionique ou non ionique à une solution de polyacrylate de sodium modifié hydrophobiquement provoque une augmentation de sa viscosité.

Le brevet US 4 432 881 décrit un milieu liquide aqueux à viscosité accrue, obtenu en dispersant dans ce milieu un polymère hydrosoluble possédant des groupes pendants hydrophobes et un agent tensioactif, utilisable tout particulièrement en récupération assistée du pétrole.

S. Evani et G. D. Rose, Proc. Am. Chem. Soc. Div. of Polym. Mater. Sci. and Eng., 1987, 57, 477 présentent deux systèmes mettant en oeuvre des associations réversibles hydrophobes pour contrôler respectivement la rhéologie de peintures contenant des latex et des fluides aqueux destinés à la récupération assistée du pétrole.

Ces deux documents montrent dans le cas des couples polymères associatifs tensioactifs concernés l'effet favorable exercé sur la viscosité du milieu soit d'une augmentation de température soit d'une augmentation de la concentration en sel dans le milieu.

Par ailleurs, de nombreuses études ont été consacrées à la détermination des interactions et des transitions de phases au sein des systèmes eau-tensioactifs en fonction de la température et de la concentration en tensioactif.

Un intérêt tout particulier a été porté aux systèmes dans lesquels le tensioactif est un tensioactif du type monoalkyléther de polyéthylèneglycol répondant à la formule

H(CH₂)ᵢ(OCH₂CH₂)ⱼOH

et noté ci-après symboliquement CᵢEⱼ où i représente le nombre d'atomes de carbone de la chaîne alkyle et j le nombre de groupements oxyde d'éthylène contenus dans la tête polaire du tensioactif.

Ces tensioactifs ont la particularité de présenter une température inférieure de démixtion : il existe une température au-dessus de laquelle la solution micellaire se sépare en deux phases, l'une diluée, l'autre concentrée en tensioactif. Cette température est appelée point de trouble. Ce phénomène est dû à une diminution de l'hydratation des groupements oxyde d'éthylène lorsque la température augmente. Par conséquent, plus la chaîne éthylèneglycol est longue, plus la température de démixtion est élevée.

Pour des températures relativement élevées et de très faibles concentrations, des phases liquides cristallines sont observées (ce qui constitue une autre spécificité de ces tensioactifs) : une phase lamellaire, noté Lₐ, qui persiste jusqu'à des taux de dilution extrêmement élevés (pour des concentrations de l'ordre de 1 % en masse pour C₁₂E₅), deux phases notées L₃ et Lₐ⁺ constituées de bicouches, et enfin plusieurs régions de coexistence liquide-liquide ont pu être mises en évidence.

Des diagrammes de phase des tensioactifs C₁₂E₃, C₁₂E₄, C₁₂E₅ sont parus dans la littérature (voir DG Hall ; JT Tiddy In Anionic Surfactants : Physical chemistry of surfactants action ; Schick, MJ Ed. ; Marcel Dekker ; New York 1987, pp. 55-108). Plus récemment, une étude consacrée au tensioactif C₁₂E₅ a été publiée (voir R. Strey, R. Schomäker, D. Roux, F. Nallet, V Olsson, Soc. Faraday Trans. 1990, 86, 2253).

Le système C₁₂E₅/H₂O a été étudié en détail, en particulier par R. Strey et al., J. Chem. Soc. Faraday Trans, 1990, 86, 2253. Il permet d'obtenir une phase lamellaire jusqu'à une teneur en eau de 99 % en masse dans une gamme étroite de température. Cette structure organisée est mise aisément en évidence par le fait que l'échantillon apparaît biréfringent entre polariseurs croisés, et peut être caractérisée plus précisément par des mesures de diffusion de rayonnement. Ainsi, des mesures de diffusion de lumière ont permis de déterminer la distance d séparant deux lamelles. Pour une fraction volumique f= 0,0125 en C₁₂E₅, d est de l'ordre de 3.10⁷ m (3000 Å). De plus, la distance de répétition entre les lamelles étant de l'ordre de grandeur des longueurs d'onde de la lumière visible, l'échantillon éclairé par de la lumière blanche apparaît coloré, à cause de la réflexion de Bragg.

La phase L₃ qui apparaît sur des diagrammes de phase est souvent appelée phase anormale ou phase éponge. Elle peut être biréfringente sous agitation et très opalescente. Ces caractéristiques seront d'autant plus marquées que la solution sera diluée. Sa structure est définie comme étant une bicouche continue à trois dimensions. En fait, les bicouches constituent une sorte de réseau connecté de façon aléatoire qui divise le volume de la solution en deux parties égales.

Par ailleurs, l'existence d'une autre phase contenant des bicouches, nommée L_{α}⁺, a été rapportée récemment par Jonströmer et Strey J. Phys. Chem., 1992, 96, 5993. Elle est observée pour des tensioactifs tels que C₁₂E₃, C₁₂E₄, C₁₄E₅, et sa structure exacte est pour l'instant inconnue. Elle a l'apparence optique de la phase L₃, c'est-à-dire qu'elle peut être biréfringente sous agitation dans certains cas, mais elle est souvent plus visqueuse que la phase L₃.

Sa zone d'existence est totalement incluse dans celle de la phase lamellaire. Les frontières entre les phases La et Lₐ⁺ sont d'ailleurs difficiles à déterminer dans la mesure où le temps de relaxation au bout duquel disparaît la biréfringence après que l'agitation a cessé peut être relativement long.

La phase Lₐ⁺ peut être actuellement définie comme une dispersion de bicouches : soit sous la forme de vésicules (simples ou multicouches), soit sous la forme d'un système diphasique où se trouvent en équilibre une phase lamellaire et une phase aqueuse. La structure reste à préciser.

La demanderesse a poursuivi des études systématiques en particulier sur les compositions aqueuses contenant des tensioactifs non ioniques et des polymères associatifs et a découvert de façon surprenante une corrélation parfaite entre les conditions de gélification de tels systèmes et celles d'apparition d'une phase correspondant à des bicouches dans le diagramme des phase du tensioactif seul en solution dans l'eau.

Cette découverte de la demanderesse l'a conduite à définir le nouveau procédé qui fait l'objet de la présente invention et qui consiste, pour obtenir, à une température donnée, une composition sous forme de gel contenant un polymère associatif, à choisir un agent tensioactif qui à cette température se trouve sous forme de bicouches en solution aqueuse.

Ainsi, selon un premier aspect, l'invention concerne un procédé de préparation d'une composition aqueuse se présentant sous forme de gel à une température donnée caractérisé en ce que l'on met en présence dans ladite composition un polymère hydrosoluble associatif constitué d'une chaîne principale hydrophile et de groupes pendants hydrophobes avec au moins un agent tensioactif se présentant sous forme de bicouches, lorsqu'il est en solution aqueuse dans les mêmes conditions de température et de concentration.

Comme on l'a vu précédemment, un certain nombre de tensioactifs ont fait l'objet d'études détaillées quant à l'aspect de leurs diagrammes de phase en solution aqueuse. Pour ces tensioactifs, on se reportera alors aux données de la littérature pour se placer dans des conditions où ils se trouvent sous forme de bicouches. Pour d'autres tensioactifs
ou des milieux aqueux contenant d'autres composants que le tensioactif susceptibles de modifier la température de transition de phases, on déterminera expérimentalement leur diagramme de phases de façon à se placer dans des conditions où les tensioactifs se trouvent sous forme de bicouches.

Bien entendu, l'homme du métier, avant même de tracer dans le détail le diagramme de phases, recherchera les zones de biréfringence qui indiquent l'emplacement d'une zone de bicouches.

Pour s'assurer de la présence des bicouches, on peut faire une étude de diffusion de rayonnements (lumière, neutrons, rayons-X,...). La microscopie électronique (cryofracture) peut être également utilisée pour confirmer la présence de vésicules et/ou de liposomes.

Selon une première variante de l'invention, les bicouches peuvent former des phases lamellaires, dispersées ou non.

Dans le cas particulier des tensioactifs de type alkyléther de polyéthylèneglycol, il apparaît, à titre d'exemple, dans la littérature que les solutions diluées de C₁₂E₄ (de 0,5 à 10 %) présentent une séparation en deux phases liquides isotropes pour des températures entre 6 et 20°C et une séparation en une phase liquide et une phase lamellaire entre 20 et 50°C. C'est dans cette gamme de températures que l'on formera avec ce type de tensioactif des compositions sous forme de gel.

Lorsque le tensioactif se présente sous forme de vésicules, telles que des liposomes, les gels selon l'invention seront obtenus par simple introduction de polymères hydrosolubles associatifs dans la dispersion de vésicules, telles que des liposomes.

A titre d'exemple de tels tensioactifs, on citera la lécithine de soja ou d'oeuf, des monoalkyléthers saturés ou insaturés de polyéthylèneglycol ou de polyglycérol linéaire ou ramifié, ou tout autre tensio-actif ionique ou non ionique susceptible de former des vésicules.

Les polymères associatifs utilisables dans le procédé de l'invention sont tous les polymères associatifs cités précédemment.

La chaîne principale hydrophile de ces polymères peut, en particulier, résulter de la polymérisation d'un monomère hydrophile comportant des fonctions permettant le greffage ultérieur de chaînes hydrophobes, par exemple des fonctions acides.

Ce mode de préparation de polymères associatifs est notamment décrit dans la publication de Shalaby W. Shalaby précédemment citée.

Il peut également s'agir d'un polymère hydrosoluble d'origine naturelle ou d'un polymère naturel rendu hydrosoluble par modification chimique.

Les polymères associatifs peuvent également résulter de la copolymérisation de monomères hydrophiles et de monomères hydrophobes. Ces monomères hydrophobes introduits dans le milieu réactionnel en beaucoup plus faible quantité que les polymères hydrophiles, comportent généralement une chaîne grasse hydrocarbonée. Pour ce mode de préparation, on pourra se reporter à la publication de S. Biggs et al. Dans J. Phys. Chem. (1992, 96, pages 1505-11).

A titre d'exemple de tels polymères, on citera les polymères acryliques, les polyéthers, les chaînes polyosidiques éventuellement partiellement substituées.

La chaîne principale hydrophile est constituée comme on l'a vu précédemment d'une succession de motifs monomères hydrophiles, et d'une fraction de monomères portant des groupements pendants très hydrophobes.

On appellera taux de modification de la chaîne hydrophile le pourcentage molaire des monomères portant des groupements pendants hydrophobes.

Ce taux est avantageusement compris entre 0,1 et 10 %, de préférence entre 0,5 et 5 %, encore plus préférentiellement entre 1 et 3 %.

Les groupements pendants hydrophobes peuvent être tout groupement pendant hydrophobe classiquement utilisé pour préparer les polymères associatifs. On choisira de préférence les groupements hydrophobes comprenant un squelette comprenant au moins 8 atomes de carbone, de préférence 12 à 18.

A titre d'exemple de tels groupements hydrophobes, on citera en particulier les chaînes hydrocarbonées linéaires, ramifiées, saturées ou non, renfermant ou non des cycles.

A titre d'exemples préférés de groupements hydrophobes, on citera les chaînes hydrocarbonées, en particulier alkyle, comprenant de 8 à 28 atomes de carbone, de préférence 12 à 18.

Les motifs modifiés sont avantageusement sous forme d'éther, d'ester ou d'amide. Ceci est en particulier le cas lorsque la chaîne principale de polymère associatif est une chaîne acrylique.

Les polymères associatifs utilisés dans le procédé de l'invention ont une masse molaire moyenne en poids comprise entre 10⁴ et 10⁷, de préférence entre 10⁵ et 10⁶.

La concentration du polymère associatif dans la composition aqueuse est généralement comprise entre 0,5 et 10 % en poids, lorsque le polymère a une masse molaire moyenne en poids de l'ordre de 150 000. Bien entendu, cette concentration pourra être plus faible pour un polymère de masse molaire moyenne plus grande.

Le tensioactif peut être tout tensioactif susceptible de se présenter sous forme de bicouches à la température et dans les conditions où l'on se propose de préparer le gel.

Il peut s'agir d'un tensioactif non ionique par exemple d'un monoalkyléther de polyéthylèneglycol de formule générale :

CᵢH₂ᵢ₊₁(OCH₂CH₂)ⱼOH

représenté symboliquement par CiEj.

On choisit alors de préférence i entre 8 et 18, de préférence égal à 12 ou 14 et j entre 1 et 10, de préférence entre 3 et 5.

Le tensioactif C₁₂E₄ présente un intérêt tout particulier selon l'invention du fait de son comportement thermique. Il peut s'agir également d'un tensioactif non ionique de formule générale :

CᵢH₂ᵢ₋₁(OCH₂CH₂)ⱼOH

qui est un monoalkyléther de polyéthylèneglycol dans lequel la chaîne alkyle comporte une insaturation. Dans ce cas, i est de préférence compris entre 12 et 24, de préférence encore i est égal à 18 ; et j est compris entre 1 et 20 et de préférence est égal à 10.

Egalement, il peut s'agir d'un monoalkyléther de polyglycérol, en particulier de formule :

R^{o}O-[C₃H₅(OH)O-]ₙ-H

dans laquelle :
i) C₃H₅(OH)O est représenté par l'une des structures suivantes prise en mélange ou séparément : 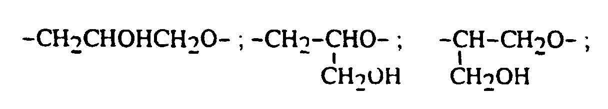
ii) n est une valeur statistique moyenne comprise entre 1 et 6 ;
iii) R^{o} représente :
   a) une chaîne aliphatique, linéaire ou ramifiée, saturée ou insaturée, contenant de 12 à 30 atomes de carbone ;
      ou un radical hydrocarboné d'un alcool de lanoline ; ou un reste d'un α-diol à longue chaîne ;
   b) un reste R¹CO, où R¹ est un radical aliphatique, linéaire ou ramifié, en C₁₁-C₁₇;
   c) un reste R²-[OC₂H₃(R³)-], où :
      * R² peut prendre la signification a) ou b) donnée pour R^{o} ;
      * OC₂H₃(R³)- est représenté par l'une des structures suivantes, prise en mélange ou séparément : 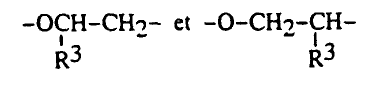
où R³ prend la signfication a) donnée pour R° ;
ou il peut encore s'agir d'un éther de polyglycérol, linéaire ou ramifié, comportant deux chaînes grasses.

Le tensioactif peut également être un tensioactif ionique.

A titre d'exemple, on citera par exemple les savons tels que l'oléate de sodium, la lécithine, telle que la lécithine de soja ou d'oeuf, les dérivés d'ammonium quaternaires tels que ceux du type dialkyldiméthylammonium, le groupement alkyle étant une chaîne grasse d'au moins 8 carbones. Il s'agit, par exemple, du bromure de dioctadécyldimé-thylammonium.

Une classe de tensioactifs particulièrement intéressants selon l'invention est constituée par ceux susceptibles de former des liposomes. A titre d'exemples de tels tensioactifs, on citera les phospholipides, la lécithine de soja ou d'oeuf.

Les compositions aqueuses contiennent de 0,1 à 10 % en poids de tensioactif ou de mélange de tensioactifs.

Afin de contrôler la formation des bicouches, il s'avère intéressant d'utiliser, non pas un tensioactif, mais un mélange de tensioactifs, en particulier un mélange de tensioactifs de la forme CᵢEⱼ. L'intérêt d'utiliser un tel mélange est de permettre d'ajuster à volonté l'hydrophobie du système. Ainsi par exemple, en remplaçant progressivement C₁₂E₅ par C₁₂E₃ tout en conservant une concentration totale constante en tensioactif, on confère au mélange eau-tensioactifs une hydrophobie apparente croissante, ce qui a pour conséquence de diminuer continûment les températures correspondant aux différentes transitions de phases.

Ainsi, l'utilisation de tels mélanges permet de faire varier les températures de transition de phase des mélanges eau-tensioactifs et en conséquence de faire varier la température d'apparition du gel en présence de polymère associatif.

Un autre moyen pour obtenir un résultat analogue est d'introduire dans la composition un alcool gras dont la fonction sera, elle aussi, de modifier la température de transition de phase. A titre d'exemple d'alcool gras utilisable dans ce but, on citera l'hexanol. Sa concentration sera avantageusement de 0,1 à 2 %.

D'autres produits peuvent également être utilisés pour contrôler la formation des bicouches. On citera tout particulièrement les stérols, les phosphates d'alcools gras ou de stérols, les sulfates d'alcools gras ou de stérols, les acides gras, les amines grasses, les saponines, les dérivés du triterpène, les céramides, les sphingosines.

Le procédé selon l'invention est utilisable, selon la nature du tensioactif:
- soit pour préparer une composition contenant un polymère hydrosoluble associatif qui soit fluide à température relativement basse et qui se gélifie dans une gamme de températures plus élevées, ce qui est le cas pour les tensioactifs dont le diagramme de phase présente une phase lamellaire dans cette gamme de températures, alors qu'il présente une zone biphasique à température plus basse. Ainsi, l'invention propose un procédé pour préparer une composition contenant un polymère hydrosoluble associatif, qui soit fluide à une température donnée dite température inférieure et se gélifie dans une gamme de températures supérieures à ladite température consistant à introduire dans ladite composition un tensioactif dont le diagramme de phase présente une zone de phase lamellaire dans ladite gamme de températures alors qu'il présente une zone biphasique à la température inférieure.
- soit pour gélifier une dispersion de vésicules, en particulier de liposomes en introduisant dans cette dispersion un polymère associatif.

Comme on l'a vu précédemment, le procédé selon l'invention fournit un moyen de faire varier considérablement la viscosité d'une composition aqueuse contenant un polymère associatif en introduisant dans ladite composition un tensioactif et en choisissant des conditions telles que ce tensioactif se trouve sous forme de bicouches.

Ce procédé pour augmenter la viscosité d'une composition aqueuse trouve une application particulièrement intéressante dans la stabilisation de suspensions aqueuses de particules, tout particulièrement dans la stabilisation des suspensions utilisées dans le domaine de la cosmétique, des peintures ou des additifs alimentaires.

Ainsi, selon un autre de ses aspects, l'invention concerne l'application du procédé précédemment décrit pour la stabilisation d'une suspension aqueuse de particules liquides ou solides.

A titre d'exemples de suspensions de particules solides qui pourront être stabilisées par le procédé de l'invention, on citera toute suspension de particules dont la dimension est comprise entre 0,02 et 50 microns, plus particulièrement des suspensions de pigments, de talcs, de micas. Ces particules peuvent éventuellement avoir subi un traitement de surface.

A titre d'exemples de suspensions de particules liquides, on citera en particulier des émulsions, tout particulièrement les émulsions de gouttelettes de dimensions comprises entre 0,05 et 10 microns, par exemple les émulsions huile dans l'eau.

A titre d'autres exemples de suspensions de particules selon l'invention, on citera les dispersions organisées sous forme de bicouches, telles que phases lamellaires, vésicules, liposomes.

Une utilisation particulièrement intéressante du procédé de l'invention est la transformation d'une suspension de liposomes en un gel par introduction dans ladite suspension d'un polymère associatif hydrosoluble. L'invention fournit ainsi un moyen de stabiliser, à toute température, une suspension de liposomes.

De telles suspensions de liposomes de viscosité accrue constituent des compositions nouvelles et par conséquent l'invention concerne également, à titre de produits nouveaux, des compositions contenant des liposomes et un polymère hydrosoluble associatif.

D'autres buts, caractéristiques et avantages de l'invention, résulteront clairement à partir de la description explicative qui va suivre faite en référence à plusieurs exemples de réalisation actuellement préférés donnés simplement à titre d'illustration. Dans les exemples tous les pourcentages sont donnés en poids sauf indication contraire.

L'invention est encore décrite en référence aux figures annexées dans lesquelles :
- la figure 1 représente des courbes avec en ordonnées la viscosité exprimée en Pa.s, en abscisse la température en °C, d'une part d'une solution à 2 % de précurseurs et d'autre part du polymère modifié 3-C18 ;
- sur la figure 2 on a représenté une courbe similaire à la figure 1 de la viscosité d'un mélange du polymère 3-C₁₂ et du tensioactif C₁₂E₄;
- la figure 3 représente le diagramme de phases d'un mélange eau-tensioactif H₂O/C₁₂E₅ (en proportions relatives en masse 98/2) et eau-tensioactif H₂O/C₁₂E₄ (en proportions relatives en masse 98/2), en proportions relatives variables en pourcentage en masse, en abscisses, à partir de 0 % en H₂O/C₁₂E₄, soit 100 % de H₂O/C₁₂E₅, jusqu'à 100 % de H₂O/C₁₂E₄, soit 0 % de H₂O/C₁₂E₅; et en fonction de la température exprimée en °C en ordonnées ;
- la figure 4 représente le diagramme de transition de phases dérivé de celui de la figure 3 par ajout de PAA-150-1-C₁₈ à 1 % en masse dans la solution ;
- la figure 5 représente les variations de viscosité avec la température de différents mélanges notés a à e exemplifiés à l'exemple 3 ;
- la figure 6 représente les variations de la viscosité en ordonnées, en Pa.s en fonction de la température en °C en abscisses pour trois mélanges exemplifiés à l'exemple 4 avec trois tensioactifs différents, pour la courbe a, le tensioactif C₁₂E₁₄, pour la courbe b, le mélange à 67,5 % de C₁₂E₄ et 32,5 % de C₁₂E₅, et pour la courbe c pour le tensioactif C₁₂E₅; et
- les figures 7, 8 et 9 donnent l'évolution du module élastique G' et du module de perte G" pour des compositions contenant respectivement 3, 2 et 1 % de lécithine de soja et différentes concentrations en polymère 1-C₁₈.

### Exemples

### Exemple 1

On prépare des polymères acryliques modifiés de structures suivantes 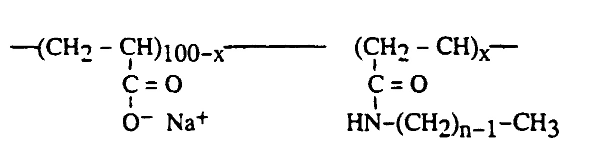 où x est le taux de modification en mole % et n le nombre d'atomes de carbone de la chaîne alkyle, à partir d'acide polyacrylique (PAA) de poids moléculaire moyen de 150 000. Les dérivés modifiés sont préparés selon le procédé décrit par T.K. Wang, I. Iliopoulos, R. Audebert, Polym. Bull. 1988, 20,577. On désignera ci-dessous les polymères modifiés symboliquement par x-Cn (à titre d'exemple 1-C18 désigne le polyacrylate modifié contenant 1 % en mole d'unités N-octadécyl-acrylamide). Les polymères modifiés ont le même degré de polymérisation que le PAA précurseur et une distribution statistique des groupes alkyles le long de la chaîne.

Les tensioactifs (C₁₂E₄, C₁₂E₅, C₁₂E₃) utilisés pour préparer les compositions sont des monododécyléthers d'oligo éthylène glycol purs à plus de 98 %.

### a) Préparation des mélanges polymère/tensioactif :

Les mélanges étudiés sont préparés en mélangeant des solutions intermédiaires du polymère d'une part, et du tensioactif d'autre part, contenant des concentrations doubles de leur concentration dans le mélange final et obtenues après 24 h d'agitation.

Dans le cas des solutions de C₁₂E₄, on prépare ces solutions à température suffisamment basse pour que le mélange soit isotrope.

### b) Contrôle de la viscosité :

Les mesures sont réalisées à l'aide d'un rhéomètre à contrainte contrôlée de type Carri Med ou pour les solutions à faible viscosité d'un appareil Low-Shear 30" de Contraves.
c) Mise en évidence de la formation d'un gel : phénomène de gélation thermique.

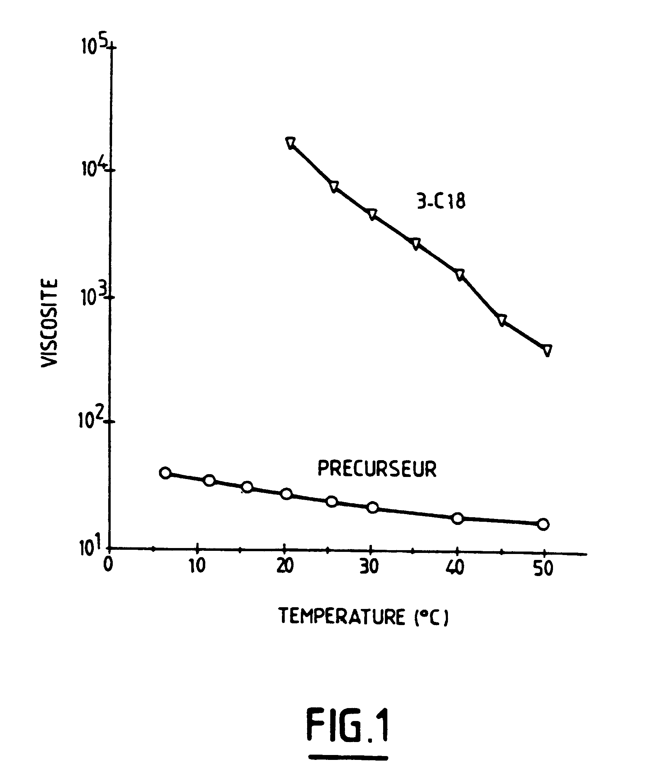

En général, la viscosité et/ou le module élastique des solutions des polymères décroît lorsqu'on élève la température. Un exemple typique d'un tel effet donné à titre comparatif est illustré sur la figure 1 qui représente la viscosité d'une solution à 2 % du précurseur et du polymère modifié 3-C18 en fonction de la température. Des comportements très différents ont été observés avec les mélanges de polymères acryliques modifiés et de C₁₂E₄ et pour des concentrations en tensioactifs supérieures à 10⁻² mol/l.

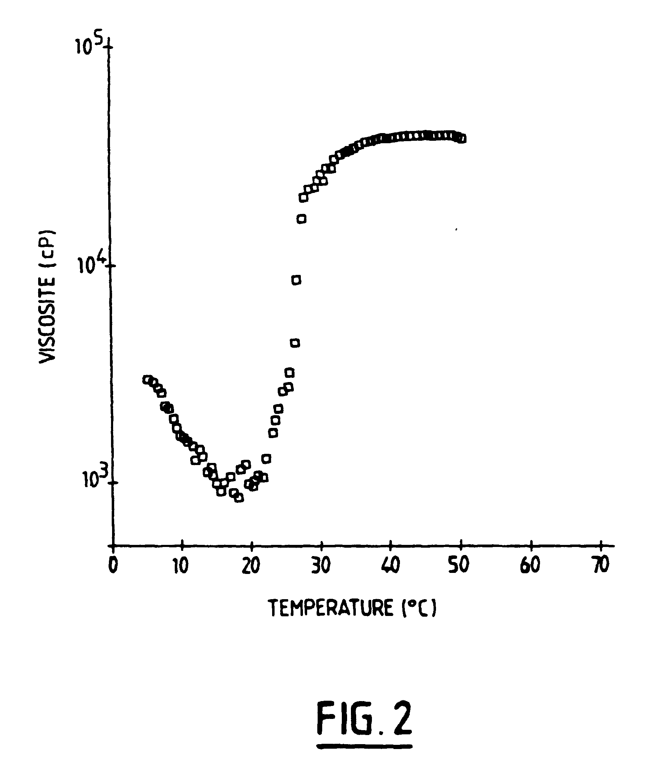

Sur la figure 2, on a représenté la viscosité des mélanges de 3-C12 et C₁₂E₄ en fonction de la température pour une concentration en tensioactif de 0,1 mole par litre et une concentration massique en polymère de 1 %. On constate des variations très nettes entre 20 et 30°C.

La viscosité peut présenter de grandes variations dans cette zone en fonction de la concentration du tensioactif.

Le changement du comportement rhéologique du système eau/tensioactif/polymère correspond à la transition de phase observée pour le système binaire tensioactif/eau : en effet, ce système passe de deux phases isotropes liquides à une température inférieure à 20°C à une dispersion lamellaire dans l'eau à une température supérieure à 20°C (cf D.J. Mitchell, G.J.T. Tiddy, L Waring, T. Bostock M.P. Mc Donald, J. Chem. Soc. Faraday Trans 1, 1981, 79, 975).

### Exemple 2

On prépare d'une part des solutions d'échantillons contenant des mélanges de C₁₂E₄ et de C₁₂E₅ en proportions variables, et d'autre part, une solution de polymère de PAA 1-C18 de masse molaire moyenne en poids de 150 000, noté ci-dessous PAR-150-1-C₁₈, à une concentration double par rapport à celle de la solution finale obtenue après mélange, de telle sorte que la composition finale dans le mélange du tensioactif soit de 2% en masse en tensioactif non ionique et dans la composition de polymère soit de 1 % en masse en polymère.

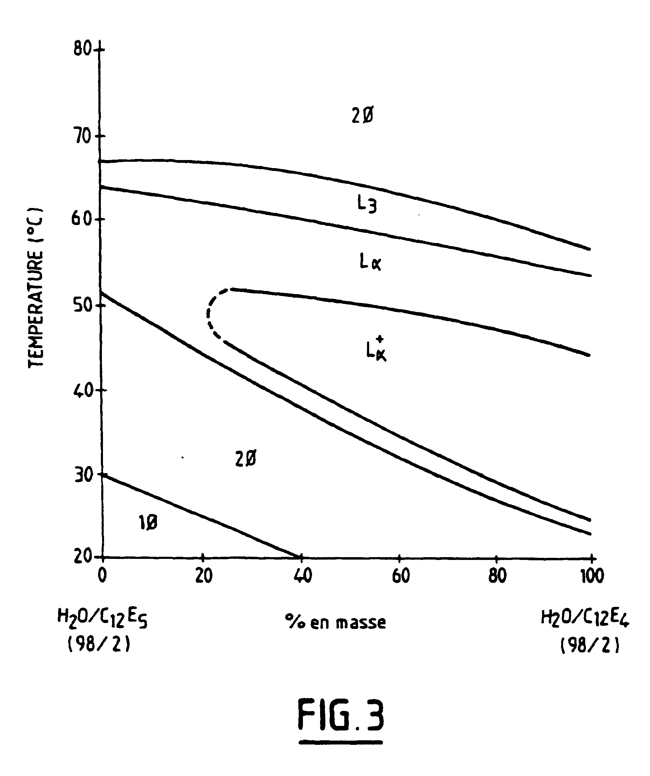

En remplaçant progressivement C₁₂E₅ par C₁₂E₄ dans le mélange, on prépare un pseudo tensioactif unique dont la température du point de trouble diminue au fur et à mesure que C₁₂E₄ devient majoritaire. Cela traduit une augmentation progressive de l'hydrophobie du pseudo tensioactif comme cela apparaît sur la figure 3.

De plus, la température de transition 2φ-Lₐ diminue elle aussi régulièrement lorsque la proportion de C₁₂E₄ augmente. Un contrôle de la température de cette transition est donc tout à fait possible puisqu'elle est régie par la composition du mélange C₁₂E₄/C₁₂E₅.

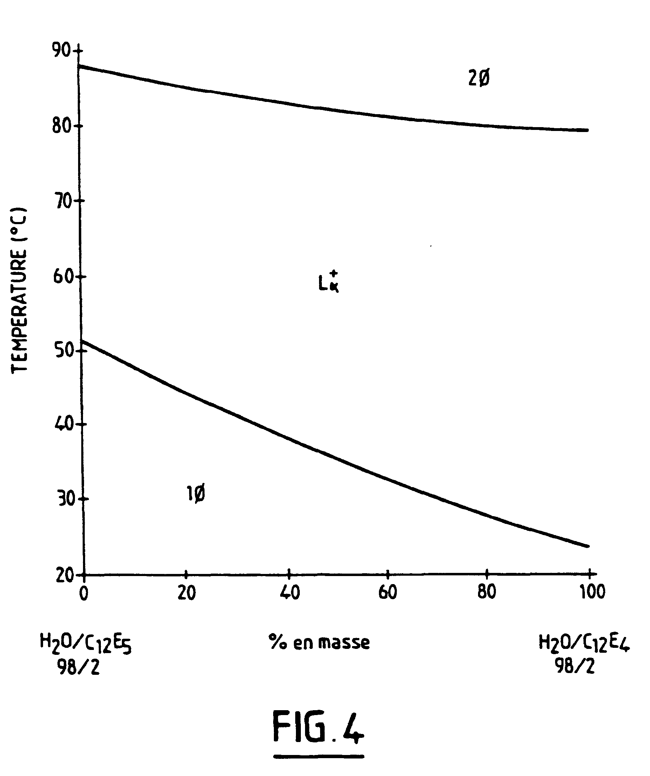

On constate sur la figure 4, qui donne le diagramme de phase du même système en présence de PAR-150-1-C₁₈, que l'ajout de PAR-150-1-C₁₈, à 1 % en masse dans la solution, modifie considérablement le diagramme de phase: la zone diphasique disparaît au profit d'une large zone monophasique isotrope limpide. Lorsque la température augmente, les échantillons deviennent biréfringents sous agitation uniquement, ce qui semble indiquer l'apparition de la phase Lₐ⁺.

La parfaite similitude entre, d'une part, la frontière séparant la zone diphasique de la zone d'existence de la phase lamellaire dans le cas des tensioactifs seuls dans l'eau et, d'autre part, celle séparant la zone uniphasique isotrope de la zone d'existence de la phase Lₐ⁺ en présence de polymère est tout à fait remarquable.

### Exemple 3

On prépare cinq échantillons notés a, b, c, d et e contenant 2 % d'agent tensioactif constitué des tensioactifs C₁₂E₄, C₁₂E₃, C₁₂E₅ dans les proportions indiquées dans le tableau 1 et 1 % de polymère noté 1-C₁₈ constitué d'acide polyacrylique de masse moléculaire moyenne 150 000 modifié à 1 % en moles par une chaîne octodécyle.

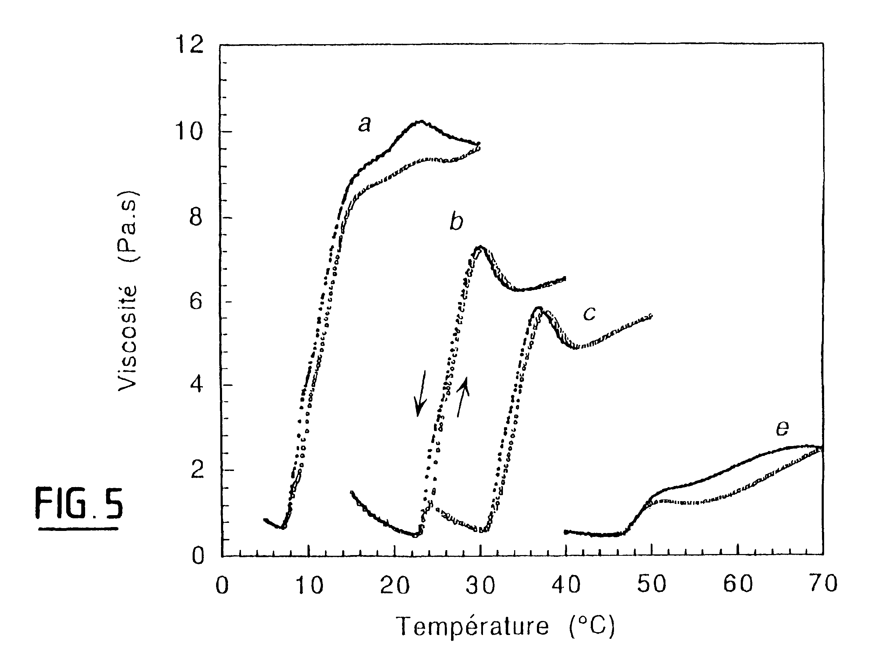

Pour chacun des mélanges, a à e, on a déterminé la température T_{L} à laquelle le système eau/tensioactif subit la transition de phase, phase isotrope/phase contenant des vésicules : cette température T_{L} figure dans le tableau 1 ci-dessous.
a) Etude de l'évolution de la viscosité
On trace, pour chaque mélange, la courbe donnant la viscosité du mélange ternaire (ou quasi-ternaire, lorsque le tensioactif est un mélange de tensioactifs) tensioactif/1-C₁₈/eau à l'aide d'un rhéomètre Carrimed à contrainte contrôlée équipé d'un cône de 4 cm de diamètre et de 2° d'angle avec un gradient de vitesse de 10 s⁻¹ et une variation de température de 0,5°C par minute. Ces courbes sont représentées sur la figure 5 sur laquelle figurent les variations de viscosité des différents mélange en montée et en descente de température.
Les températures T_{G} de gélification des différents systèmes déterminés à partir des courbes de la figure 5 sont indiquées dans le tableau 1 ci-dessous.

**TABLEAU 1**

| Courbe | Composition | Température de la transition de phases T_{L} | Température de gélification T_{G} |
|---|---|---|---|
| a | C₁₂E₄ 56.5 % C₁₂E₃ 43.5% | 7,5°C | 7,5°C |
| b | C₁₂E₄ | 23°C | 23°C |
| c | C₁₂E₄ 65,8 % C₁₂E₅ 34,2 % | 32°C | 31,5°C |
| e | C₁₂E₅ | 51°C | 48°C |

b) Etude des courbes d'écoulement
Par ailleurs, pour chacun des échantillons ci-dessus, on a tracé des courbes d'écoulement à différentes températures, représentant les variations de viscosité en fonction du gradient de vitesse à l'aide de l'appareil Rheometrics qui réalise des balayages logarithmiques en gradient de vitesse aller et retour (encore appelés boucles thixotropiques) de 0,1s⁻¹ à 100 s⁻¹. Deux vitesses de balayage ont été utilisées :
- montée et descente en gradient effectuées chacune en 14 minutes, séparées par un palier d'une durée de 30 secondes à 100 s⁻¹.
- montée et descente en gradient effectuées chacune en 1 heure, séparées par un palier d'une durée de 1 minute à 100 s⁻¹

On a observé une nette modification de l'allure de ces courbes d'écoulement lorsque la température de transition de phase est franchie.
c) Etude en oscillation
Les échantillons précédents ont été étudiés en régime dynamique, à l'aide de l'appareil Rheometrics qui applique à l'échantillon une déformation sinusoïdale d'amplitude fixée par l'opérateur qui mesure simultanément la contraite sinusoïdale qui en résulte et en déduit, après calcul, les valeurs du module élastique G' et du module de perte G".
Tous les échantillons étudiés ont sensiblement le même domaine linéaire qui s'étend de 0 à 100 % de déformation. Afin de les comparer, on les soumet à une déformation sinusoïdale d'amplitude 8 % et on a étudié l'évolution de G' et G" sur une gamme de fréquence de 0,015 Hz à 15 Hz, à différentes températures.

Chaque échantillon a été soumis au protocole suivant :
- mesure de G' et G" sans précisaillement
- mesure de G' et G" après avoir soumis l'échantillon à un gradient de vitesse de 10 s⁻¹ pendant 10 secondes
- mesure de G' et G" après avoir soumis l'échantillon à un gradient de vitesse de 30 s⁻¹ pendant 10 secondes
- mesure de G' et G" après avoir soumis l'échantillon à un gradient de vitesse de 50 s⁻¹ pendant 10 secondes
- mesure de G' et G" après avoir soumis l'échantillon à un gradient de vitesse de 30 s⁻¹ pendant 30 secondes.

Les résultats obtenus montrent qu'à une température supérieure à T_{G}, l'évolution des modules en fonction de la fréquence varie peu d'un échantillon à l'autre. Cette similitude au niveau macroscopique est probablement liée à une analogie de structure au niveau microscopique. Les valeurs des modules sont fonction des tensioactifs contenus dans la solution.

Avant la transition, l'allure des courbes des modules dépend peu de l'échantillon considéré : une droite de pente voisine de 2 pour G', une droite de pente voisine de 1 pour G". De plus, ces droites deviennent superposables par translation simple le long de l'axe des fréquences.

### Exemple 4 : Mise en évidence des variations de viscosité avec la température.

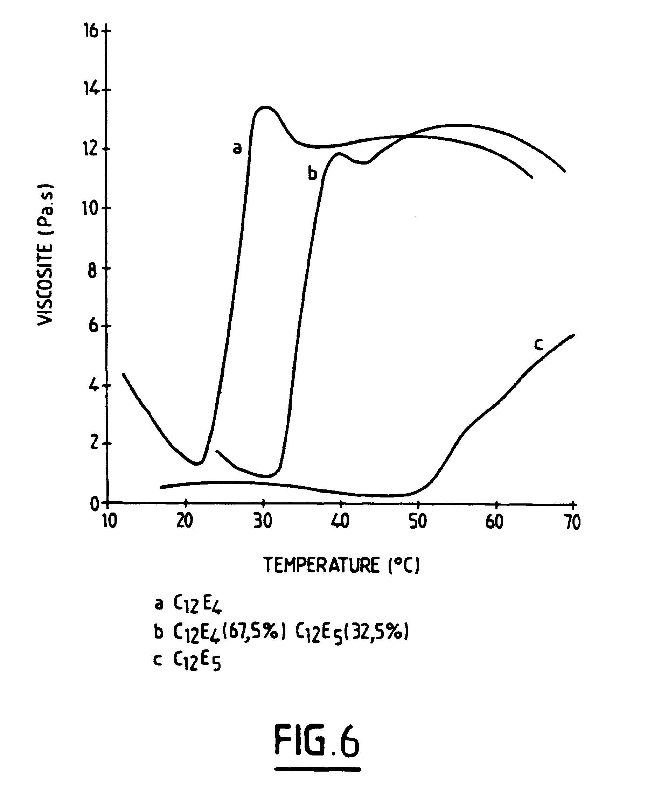

On a représenté sur la figure 6 les variations de la viscosité en Pa.s avec la température en °C pour une solution d'acide polyacrylique modifié noté PAA-150-1-C₁₈ à 1 % défini à l'exemple 2 et différents tensioactifs ou mélange de tensioactifs pour une concentration totale en tensioactif de 2 % en masse.

Les courbes a, b, c, de la figure 6, correspondent respectivement aux tensioactifs suivants :
- C₁₂E₄ pour la courbe a,
- mélange à 67,5 % de C₁₂E₄ et 32,5 % de C₁₂E₅ pour la courbé b,
- C₁₂E₅ pour la courbe c.

Une augmentation de la viscosité est observée à la température de la transition entre la phase isotrope et la phase Lₐ⁺ du mélange tensioactif/polymère. Cette augmentation est brutale et importante (viscosité apparente multipliée par 10) pour les échantillons contenant exclusivement et majoritairement C₁₂E₄. Le phénomène observé est légèrement différent pour les échantillons contenant exclusivement et majoritairement C₁₂E₅: l'augmentation de viscosité est plus progressive et de moindre importance (viscosité multipliée par un facteur de l'ordre de 5).

Ce phénomène est reproductible quant à la température à laquelle apparaît la modification de viscosité, lorsque l'expérience est répétée, en montée et en descente en température.

### Exemple 5 : Mélanges contenant 4 % de tensioactifs et 1 % de polymère 1-C₁₈

On a étudié, comme dans l'exemple 3, le comportement rhéologique de solutions contenant cette fois 4 % de C₁₂E₄ et 1 % de polymère 1-C₁₈ en traçant des boucles thixotropiques à différentes températures et en examinant l'évolution des modules en fonction de la fréquence.

### a) Courbes d'écoulement

Avant la transition de phases, la viscosité de l'échantillon ne varie pratiquement pas avec le gradient de vitesse et elle est plus faible (d'un ordre de grandeur environ) que la viscosité de la solution à 2 % en C₁₂E₄ et 1 % en polymère 1-C₁₈ dans les mêmes conditions.

Après la transition, la viscosité diminue régulièrement avec le gradient de vitesse, mais prend des valeurs plus élevées que celles de la solution à 2 % en C₁₂E₄.

Dans la solution contenant 4 % de C₁₂E₄, avant la température de transition, les micelles sont plus nombreuses que dans la solution à 2%. Le rapport agrégats/chaînes macromoléculaires est augmenté. Cela permet de diminuer les associations entre macromolécules qui pouvaient persister dans la solution à 2 % en tensioactif.

Au-delà de la température de transition T_{G}, la concentration plus élevée en tensioactif se traduit par une augmentation du nombre et de la taille des vésicules. Ceci conduit à un nombre de pontage entre chaînes plus élevé.

### b) Etude en oscillation

On observe un comportement analogue à celui des solutions contenant 2 % de tensioactif non ionique et 1 % de polymère.

Avant la transition de phases, le module élastique G' est inférieur au module de perte G". En coordonnées logarithmiques, les courbes représentatives de G' et G" sont des droites de pente voisine de 1 et, à fréquence égale, leurs valeurs sont plus faibles que celles de la solution à 2 % en C₁₂E₄ et 1 % en polymère. Au-delà de la transition, le module élastique devient supérieur au module de perte et tous deux varient peu en fonction de la fréquence.

### c) Influence d'un ajout de 30 % en masse de glycérine

Le comportement rhéologique n'est pas modifié. Seule la température de la transition est modifiée et la valeur de la viscosité est légèrement augmentée.

### Exemple 6 : Mélange contenant 10 % en tensioactif et du polymère 1-C₁₈

### a) Etude de l'évolution de la viscosité en fonction de la température

On procède comme à l'exemple 3 et on effectue plusieurs fois de suite la montée et la descente en température, à une vitesse de 0,5°C par minute, à un gradient de vitesse de 10 s⁻¹ avec des compositions contenant 1, 2, 3 et 5 % de polymère 1-C₁₈ et 10 % de C₁₂E₄.

L'agmentation de viscosité se produit toujours à la même température (23°C ± 1°C) qui est la température de la transition de phases (phase micellaire à phase contenant des bicouches).

Ces systèmes atteignent leur état d'équilibre plus lentement que les échantillons ne contenant que 2 % en tensioactif.

### b) Etude en oscillation

Au-delà de la température de transition, les échantillons ont un comportement de solide élastique : le module élastique G' et le module de perte G" varient peu en fonction de la fréquence et G' est supérieur à G".

### c) Influence de l'ajout de 30 % de glycérine

De la même façon que pour les échantillons contenant 4 % de C₁₂E₄, le comportement des solutions n'est pas modifié ; seules les températures des transitions de phases sont modifiées.

### Exemple 7 : Composition contenant des liposomes

On prépare une suspension de liposomes dans l'eau à partir de
- 4 % de lécithine de soja (Alcolec F100)
- 96 % d'eau.

Le mélange est homogénéisé aux ultrasons pendant 15 min à 4°C avec une puissance de 150 W.

On obtient une suspension de liposomes de viscosité voisine de celle de l'eau.

On introduit dans cette suspension de liposomes un volume égal de solution de polymère associatif désigné par PAR-150-3-C₁₈ et constitué de polyacide acrylique de poids moléculaire moyen de 150 000 contenant 3 % en moles d'unité N-octadécylacrylamide à des concentrations massiques de 1 et 2 %. On observe dans les deux cas une très nette augmentation de viscosité.

### Exemple 8 : Composition contenant des liposomes

On prépare une suspension aqueuse contenant 2 % en masse de liposomes obtenus à partir de lécithine de soja et de β-sitostérol pris dans un rapport pondéral de 9/1.

On ajoute, comme dans le cas précédent, un volume égal de solution à 1 % et 2 % de PAA-150-3-C₁₈: dans les deux cas on observe la formation d'un gel.

### Exemple 9 : Composition contenant des liposomes

On prépare trois suspensions A, B, C de liposomes contenant respectivement 3 %, 2 % et 1 % de lécithine de soja (Alcolec F100).

Les solutions ainsi obtenues ont ensuite été microfluidisées. La taille des particules a été déterminée en examinant les échantillons en microscopie électronique après cryofracture : elle varie entre 50 nm et 500 nm.

L'ajout de polymère 1-C₁₈ à ces solutions provoque une augmentation de viscosité en fonction des concentrations respectives en phospholipides et en polymère. Quelle que soit la températur considérée, les phospholipides s'organisent en bicouches. Le paramètre qui conditionne le comportement rhéologique des solutions n'est donc plus la température, mais les teneurs respectives en phospholipides et en polymère 1-C₁₈, et, le rapport entre ces deux concentrations.

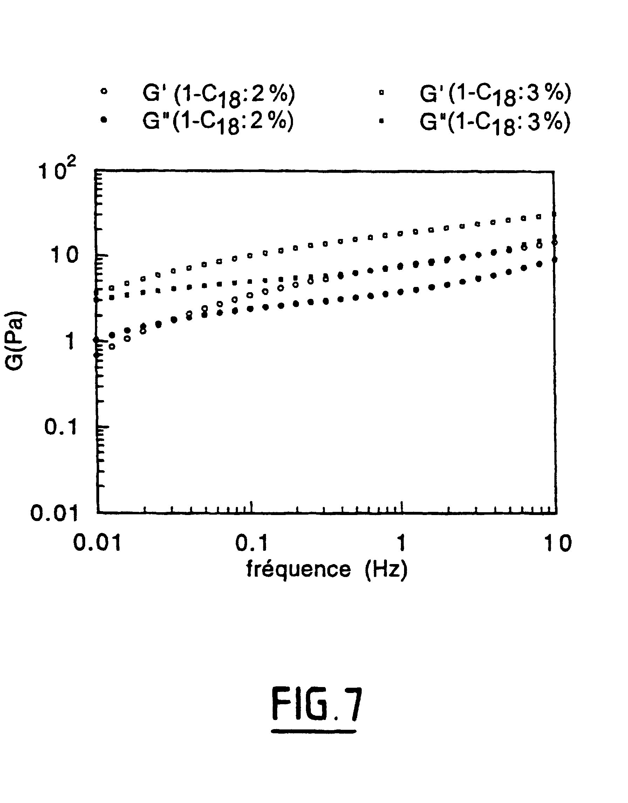

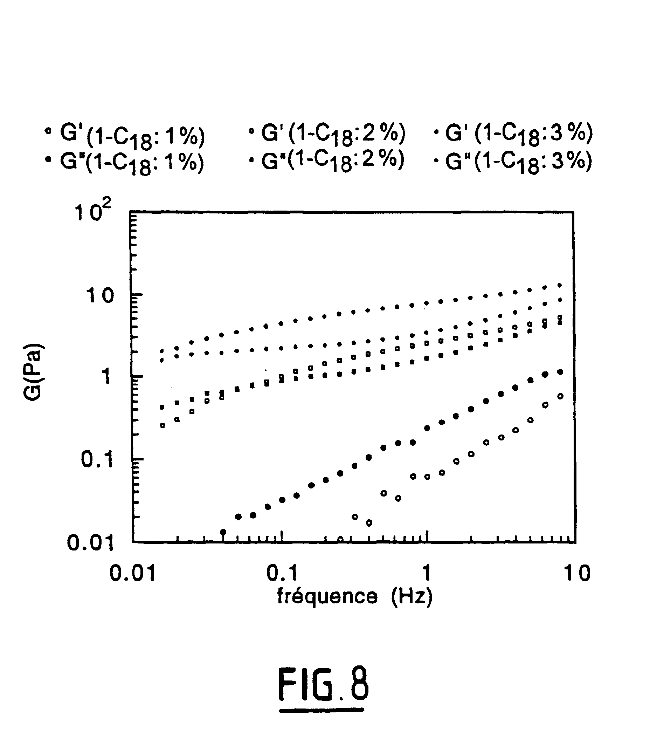

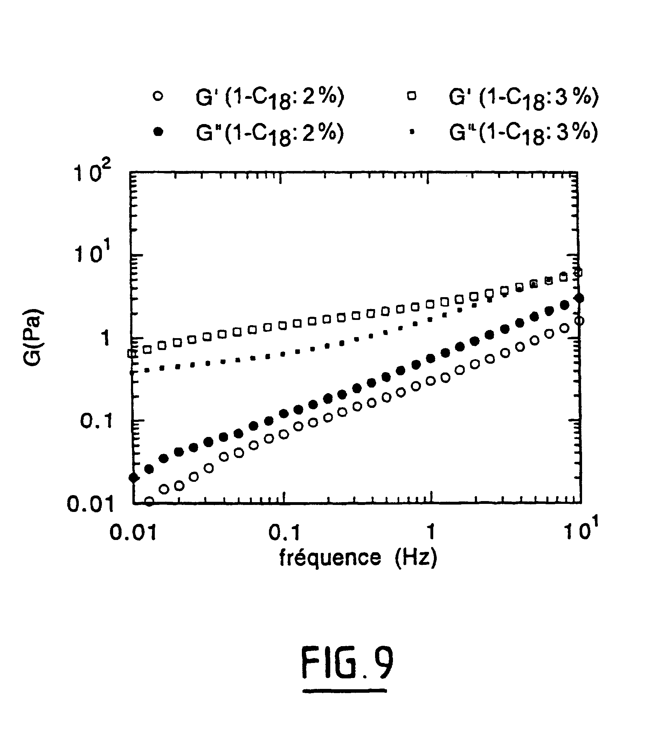

On a étudié l'évolution du module élastique G' et du module de perte G" de solution d'Alcolec en fonction des teneurs respectives en phospholipides et en polymère 1-C₁₈. Les résultats sont donnés sur les courbes des figures 7, 8, 9 qui donnent, pour chacune, des compositions A, B et C l'évolution des modules G' et G" en fonction de la fréquence pour les différentes concentrations en 1-C₁₈.

### Exemple 10 : Composition contenant des vésicules lipidiques non ioniques

On prépare une suspension dans l'eau de vésicules lipidiques non ioniques constituée de l'octadécyléther de polyéthylèneglycol de formule CH₃(CH₂)₇CH=CH(CH₂)₇CH₂(OCH₂CH₂)ₙOH disponible dans le commerce sous le non de Brij 96-A7606. On introduit dans l'eau 4 % en poids de ce tensio-actif et l'on chauffe à 50°C ce mélange sous agitation, puis refroidissement à température ambiante.

On mélange cette suspension en proportion 1/1 avec une solution à 2 % en poids du polymère associatif précédemment désigné par PAA-150-3C18.

On observe une nette augmentation de la viscosité et la formation d'un gel.

## Revendications

1. Procédé de préparation d'une composition aqueuse se présentant sous forme d'un gel à une température donnée, **caractérisé en ce qu'**il comprend les étapes de :
a) sélection d'au moins un agent tensioactif se présentant sous forme de bicouches lorsqu'il est en solution aqueuse à une concentration comprise entre 0,1 et 10 % en poids dans ladite solution aqueuse à ladite température donnée, et
b) préparation d'une composition aqueuse comprenant ledit tensioactif à ladite concentration de 0,1 à 10 % en poids et une quantité efficace pour former un gel à ladite température donnée d'un polymère hydrosoluble associatif ayant une masse molaire moyenne en poids comprise entre 10⁴ et 10⁷, ledit polymère associatif étant constitué d'une chaîne principale hydrophile et de groupes pendants hydrophobes.

2. Procédé selon la revendication 1, **caractérisé en ce que** lesdites bicouches sont formées de phases lamellaires dispersées ou non.

3. Procédé selon la revendication 1, **caractérisé en ce que** lesdites bicouches forment des vésicules telles que des liposomes.

4. Procédé pour préparer une composition contenant un polymère hydrosoluble associatif tel que défini dans la revendication 1, qui soit fluide à une première température dite température inférieure et se gélifie dans une gamme de températures supérieures à ladite première température, consistant à introduire dans ladite composition, de 0,1 à 10 % en poids d'un tensioactif dont le diagramme de phase présente une zone de phase lamellaire dans ladite gamme de températures alors qu'il présente une zone biphasique à la température inférieure.

5. Procédé selon l'une des revendications 1 à 4, **caractérisé en ce que** ladite chaîne principale hydrophile résulte de la polymérisation d'un monomère hydrophile ou est constituée d'un polymère hydrosoluble d'origine naturelle ou d'un polymère naturel rendu hydrosoluble par modification chimique.

6. Procédé selon l'une des revendications 1 à 5, **caractérisé en ce que** ladite chaîne principale hydrophile est une chaîne acrylique, un polyéther ou une chaîne polyosidique éventuellement partiellement substituée.

7. Procédé selon l'une des revendications 1 à 6, **caractérisé en ce que** le taux de modification de ladite chaîne hydrophile, défini comme étant le pourcentage de groupements monomères hydrophiles portant des groupements hydrophobes est compris entre 0,1 et 10 %, de préférence entre 0,5 et 5 %, plus préférentiellement entre 1 et 3 %.

8. Procédé selon l'une des revendications 1 à 7, **caractérisé en ce que** les groupements hydrophobes comprennent un squelette carboné comprenant au moins 8 atomes de carbone.

9. Procédé selon la revendication 8, **caractérisé en ce que** les groupements hydrophobes sont des chaînes hydrocarbonées, en particulier alkyles comprenant de 8 à 28 atomes de carbone, de préférence de 12 à 18.

10. Procédé selon l'une des revendications 1 à 9, **caractérisé en ce que** les groupements hydrophobes sont liés à la chaîne principale par des groupements éther, ester ou amide.

11. Procédé selon l'une des revendications 1 à 10, **caractérisé en ce que** la concentration du polymère associatif est comprise entre 0,5 et 10 % en poids lorsque ledit polymère a une masse molaire moyenne en poids de l'ordre de 150 000, cette concentration étant plus faible pour une masse moyenne molaire plus forte.

12. Procédé selon l'une des revendications 1 à 11, **caractérisé en ce que** ledit agent tensioactif est un agent tensioactif non ionique ou ionique.

13. Procédé selon l'une des revendications 1 à 12, **caractérisé en ce que** l'agent tensioactif est choisi dans le groupe constitué des monoalkyléthers de polyéthylèneglycol de formule générale :
CᵢH₂ᵢ₊₁(OCH₂CH₂)ⱼOH
avec i compris entre 8 et 18, de préférence égal à 12 ou 14, et j compris entre 1 et 10, de préférence entre 3 et 5.

14. Procédé selon l'une des revendications 1 à 12, **caractérisé en ce que** l'agent tensioactif est choisi dans le groupe constitué des monoalkyléthers de polyglycérol, en particulier de formule :
R^{o}O-[C₃H₅(OH)O-]ₙ-H
dans laquelle :
i) C₃H₅(OH)O est représenté par l'une des structures suivantes prise en mélange ou séparément : 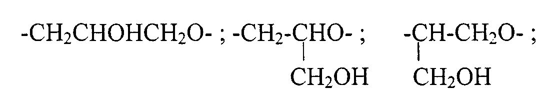
ii) n est une valeur statique moyenne comprise entre 1 et 6 ;
iii) R^{o} représente :
a) une chaîne aliphatique, linéaire ou ramifiée, saturée ou insaturée, contenant de 12 à 30 atomes de carbone ; ou un radical hydrocarboné d'un alcool de lanoline ; ou un reste d'un α-diol à longue chaîne ;
b) un reste R¹CO, où R¹ est un radical aliphatique, linéaire ou ramifié, en C₁₁-C₁₇;
c) un reste R²-[OC₂H₃(R³)-], où :
* R² peut prendre la signification a) ou b) donnée pour R^{o} ;
* OC₂H₃(R³)- est représenté par l'une des structures suivantes, prise en mélange ou séparément : 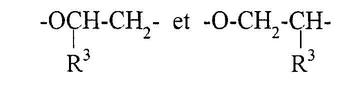
où R³ prend la signfication a) donnée pour R^{o};
ou il peut encore s'agir d'un éther de polyglycérol, linéaire ou ramifié, comportant deux chaînes grasses.

15. Procédé selon l'une des revendications 1 à 12, **caractérisé en ce que** l'agent tensioactif est un agent tensioactif ionique tel qu'un savon, par exemple l'oléate de sodium, de la lécithine, telle que la lécithine de soja ou d'oeuf, un dérivé d'ammonium quaternaire, tel que du type dialkyldiméthylammonium, le groupement aklyle étant une chaîne grasse d'au moins 8 atomes de carbone, par exemple le bromure de dioectadécyldiméthylammonium.

16. Procédé selon l'une des revendications 1 à 15, **caractérisé en ce que** l'agent tensioactif est susceptible de former des liposomes, des vésicules ou des phases lamellaires.

17. Procédé selon l'une des revendications 1 à 16, **caractérisé en ce que** la composition contient en outre un composé destiné à contrôler la formation des bicouches, par exemple un deuxième tensioactif, un alcool gras, un stérol, un phosphate d'alcool gras ou de stérol, un sulfate d'alcool gras ou de stérol, un acide gras, une amine grasse, une saponine, un dérivé de triterpène, une céramide, une sphingosine.

18. Utilisation du procédé selon l'une des revendications 1 à 17 pour stabiliser, par augmentation de sa viscosité, une suspension aqueuse de particules liquides ou solides.

19. Utilisation selon la revendication 18, **caractérisée en ce que** lesdites particules sont constituées de gouttelettes de liquide non miscible à l'eau de dimension comprise entre 0,05 et 10 microns et qu'il s'agit par exemple d'une émulsion huile dans eau.

20. Utilisation selon la revendication 18, **caractérisée en ce que** lesdites particules sont des particules solides, en particulier des pigments ou des talcs, ou des micas, éventuellement traités en surface, de dimension comprise entre 0,02 et 50 microns.

21. Utilisation selon la revendication 18, **caractérisée en ce que** ladite suspension est une dispersion organisée de bicouches telle qu'une phase lamellaire, de vésicules ou de liposomes.

22. Utilisation selon l'une quelconque des revendications 18 à 21, **caractérisée en ce qu'**elle est mise en oeuvre pour la préparation d'une composition cosmétique.

23. Procédé pour stabiliser une suspension de vésicules par augmentation de sa viscosité, notamment une suspension de liposomes, **caractérisé en ce qu'**il consiste à ajouter à ladite suspension un polymère associatif tel que défini dans l'une des revendications précédentes.

## Patentansprüche

1. Verfahren zur Herstellung einer wässerigen Zusammensetzung, die bei einer gegebenen Temperatur in Form eines Gels vorliegt, **dadurch gekennzeichnet, dass** es folgende Schritte umfasst:
a) Auswählen mindestens eines grenzflächenaktiven Stoffs, der in wässeriger Lösung in einer Konzentration zwischen 0,1 und 10 Gew.% in dieser wässerigen Lösung bei der gegebenen Temperatur in Form von Doppelschichten vorliegt, und
b) Herstellen einer wässerigen Zusammensetzung, die den grenzflächenaktiven Stoff in einer Konzentration von 0,1 bis 10 Gew.% und eine wirksame Menge eines assoziativen wasserlöslichen Polymers mit einer mittleren Molmasse in Gewicht zwischen 10⁴ und 10⁷ enthält, um bei der gegebenen Temperatur ein Gel zu bilden, welches assoziative Polymer aus einer hydrophilen Hauptkette und hydrophoben Nebengruppen besteht.

2. Verfahren nach Anspruch 1, **dadurch gekennzeichnet, dass** die Doppelschichten aus gegebenenfalls dispergierten lamellaren Phasen gebildet sind.

3. Verfahren nach Anspruch 1, **dadurch gekennzeichnet, dass** die Doppelschichten Vesikel wie Liposome bilden.

4. Verfahren zur Herstellung einer Zusammensetzung, die ein assoziatives wasserlösliches Polymer nach Anspruch 1 enthält, das bei einer ersten Temperatur, genannt niedrigere Temperatur, fluid ist und in einem höheren Temperaturbereich als die erste Temperatur geliert, welches Verfahren darin besteht, dass in die Zusammensetzung 0,1 bis 10 Gew.% eines grenzflächenaktiven Stoffs eingebracht werden, dessen Phasendiagramm eine Zone lamellarer Phase in besagtem Temperaturbereich aufweist, während es eine Zweiphasenzone bei der niedrigeren Temperatur aufweist.

5. Verfahren nach einem der Ansprüche 1 bis 4, **dadurch gekennzeichnet, dass** die hydrophile Hauptkette aus der Polymerisation eines hydrophilen Monomers resultiert oder durch ein wasserlösliches Polymer natürlichen Ursprungs oder ein natürliches Polymer, das durch chemische Modifikation wasserlöslich gemacht wurde, gebildet ist.

6. Verfahren nach einem der Ansprüche 1 bis 5, **dadurch gekennzeichnet, dass** die hydrophile Hauptkette eine Acrylkette, ein Polyether oder eine gegebenenfalls teilweise substituierte Polyosidkette ist.

7. Verfahren nach einem der Ansprüche 1 bis 6, **dadurch gekennzeichnet, dass** der Modifikationsgrad der hydrophilen Kette, definiert als Prozentsatz an hydrophilen monomeren Gruppen, die hydrophobe Gruppen tragen, zwischen 0,1 und 10 %, vorzugsweise zwischen 0,5 und 5 %, bevorzugter zwischen 1 und 3 % beträgt.

8. Verfahren nach einem der Ansprüche 1 bis 7, **dadurch gekennzeichnet, dass** die hydrophoben Gruppen ein Kohlenstoffskelett mit mindestens 8 Kohlenstoffatomen aufweisen.

9. Verfahren nach Anspruch 8, **dadurch gekennzeichnet, dass** die hydrophoben Gruppen Kohlenwasserstoffketten, insbesondere Alkyle mit 8 bis 28, vorzugsweise 12 bis 18 Kohlenstoffatomen sind.

10. Verfahren nach einem der Ansprüche 1 bis 9, **dadurch gekennzeichnet, dass** die hydrophoben Gruppen über Ether-, Ester- oder Amidgruppen mit der Hauptkette verbunden sind.

11. Verfahren nach einem der Ansprüche 1 bis 10, **dadurch gekennzeichnet, dass** die Konzentration des assoziativen Polymers zwischen 0,5 und 10 Gew.% beträgt, wenn das Polymer eine mittlere Molmasse in Gewicht in der Größenordnung von 150 000 aufweist, wobei diese Konzentration bei einer höheren mittleren Molmasse geringer ist.

12. Verfahren nach einem der Ansprüche 1 bis 11, **dadurch gekennzeichnet, dass** das grenzflächenaktive Mittel ein nichtionisches oder ein ionisches grenzflächenaktives Mittel ist.

13. Verfahren nach einem der Ansprüche 1 bis 12, **dadurch gekennzeichnet, dass** das grenzflächenaktive Mittel ausgewählt ist aus der Gruppe bestehend aus Polyethylenglykolmonoalkylethern der allgemeinen Formel:
CᵢH₂ᵢ₊₁(OCH₂CH₂)ⱼOH,
wobei i zwischen 8 und 18, vorzugsweise 12 oder 14, und j zwischen 1 und 10, vorzugsweise zwischen 3 und 5, beträgt.

14. Verfahren nach einem der Ansprüche 1 bis 12, **dadurch gekennzeichnet, dass** das grenzflächenaktive Mittel ausgewählt ist aus der Gruppe bestehend aus Polyglycerinmonoalkylethern, insbesondere der Formel:
R⁰O-[C₃H₅(OH)O-]ₙ-H,
worin
i) C₃H₅(OH)O durch eine der folgenden Strukturen, gemischt oder getrennt, dargestellt wird: 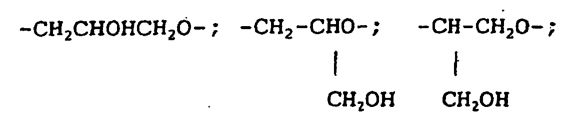
ii) n ein mittlerer statischer Wert zwischen 1 und 6 ist;
iii) R^{o} bedeutet:
a) eine lineare oder verzweigte, gesättigte oder ungesättigte aliphatische Kette mit 12 bis 30 Kohlenstoffatomen; oder einen Kohlenwasserstoffrest eines Lanolinalkohols; oder einen Rest eines langkettigen α-Diols;
b) einen Rest R¹CO, worin R¹ ein linearer oder verzweigter aliphatischer C₁₁-C₁₇-Rest ist;
c) einen Rest R²-[OC₂H₃(R³)-], worin
* R² die für R^{o} angegebene Bedeutung a) oder b) haben kann;
* OC₂H₃(R³)- durch eine der folgenden Strukturen, gemischt oder getrennt, dargestellt wird: 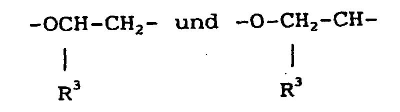
worin R³ die für R^{o} angegebene Bedeutung a) hat;
oder es sich auch noch um einen linearen oder verzweigten Polyglycerinether mit zwei Fettsäureketten handeln kann.

15. Verfahren nach einem der Ansprüche 1 bis 12, **dadurch gekennzeichnet, dass** der grenzflächenaktive Stoff ein ionischer grenzflächenaktiver Stoff wie eine Seife ist, beispielsweise Natriumoleat, Lecithin wie Soja- oder Eilecithin, ein quaternäres Ammoniumderivat wie vom Typ Dialykldimethylammonium, wobei die Alkylgruppe eine Fettsäuregruppe mit mindestens 8 Kohlenstoffatomen, beispielsweise Dioctadecyldimethylammoniumbromid, ist.

16. Verfahren nach einem der Ansprüche 1 bis 15, **dadurch gekennzeichnet, dass** der grenzflächenaktive Stoff Liposome, Vesikel oder lamallare Phasen bilden kann.

17. Verfahren nach einem der Ansprüche 1 bis 16, **dadurch gekennzeichnet, dass** die Zusammensetzung weiters eine Verbindung zur Steuerung der Bildung der Doppelschichten enthält, beispielsweise einen zweiten grenzflächenaktiven Stoff, einen Fettalkohol, ein Sterin, ein Fettalkohol- oder Sterinphosphat, ein Fettalkohol- oder Sterinsulfat, eine Fettsäure, ein Fettamin, ein Saponin, ein Triterpenderivat, ein Ceramid, ein Sphingosin.

18. Verwendung des Verfahrens nach einem der Ansprüche 1 bis 17 zur Stabilisierung einer wässerigen Suspension von flüssigen oder festen Teilchen durch Erhöhung ihrer Viskosität.

19. Verwendung nach Anspruch 18, **dadurch gekennzeichnet, dass** die Teilchen aus flüssigen, mit Wasser nicht mischbaren Tröpfchen einer Größe zwischen 0,05 und 10 µm bestehen und es sich beispielsweise um eine Öl-in-Wasser-Emulsion handelt.

20. Verwendung nach Anspruch 18, **dadurch gekennzeichnet, dass** die Teilchen gegebenenfalls oberflächenbehandelte feste Teilchen, insbesondere Pigmente oder Talke, oder Glimmer, einer Größe zwischen 0,02 und 50 µm sind.

21. Verwendung nach Anspruch 18, **dadurch gekennzeichnet, dass** die Suspension eine organisierte Dispersion von Doppelschichten ist, wie eine lamellare Phase, eine Vesikel-Phase oder eine Liposomen-Phase.

22. Verwendung nach einem der Ansprüche 18 bis 21, **dadurch gekennzeichnet, dass** sie zur Herstellung einer kosmetischen Zusammensetzung eingesetzt wird.

23. Verfahren zur Stabilisierung einer Vesikel-Suspension, insbesondere eine Liposomen-Suspension, durch Erhöhung ihrer Viskosität, **dadurch gekennzeichnet, dass** es darin besteht, dass der Suspension ein assoziatives Polymer nach einem der vorhergehenden Ansprüche zugesetzt wird.

## Claims

1. A process for preparing an aqueous composition in the form of a gel at a given temperature, **characterised in that** it comprises the steps of:
a) selection of at least one surfactant, which is under the form of bilayers, when it is in aqueous solution at a concentration comprised between 0.1 and 10 % by weight in said aqueous solution at said given temperature, and
b) preparation of an aqueous composition comprising said surfactant at said concentration of 0.1 to 10 % by weight and an effective amount, for forming a gel at said given temperature, of an associative water-soluble polymer having a mass average molar mass comprised between 10⁴ and 10⁷, said associative polymer being constituted by a hydrophilic main chain and hydrophobic pendant groups.

2. The process according to claim 1, **characterised in that** said bilayers are formed of lamellar phases which may or may not be dispersed.

3. The process according to claim 1, **characterised in that** said bilayers form vesicles such as liposomes.

4. A process for preparing a composition containing an associative water-soluble polymer, as defined in claim 1, which is fluid at a given first temperature termed the lower temperature and which gels within a range of temperatures which are higher than said first temperature, consisting in introducing into said composition from 0.1 to 10 % by weight of a surfactant having a phase diagram which exhibits a lamellar phase zone in said range of temperatures and a biphase zone at the lower temperature.

5. The process according to one of claims 1 to 4, **characterised in that** said hydrophilic main chain results from the polymerisation of a hydrophilic monomer or is constituted by a water-soluble polymer of natural origin or a natural polymer rendered water-soluble by chemical modification.

6. The process according to one of claims 1 to 5, **characterised in that** said hydrophilic main chain is an acrylic chain, a polyether or a polyosidic chain which may be partially substituted.

7. The process according to one of claims 1 to 6, **characterised in that** the modification percentage of said hydrophilic chain, defined as the percentage of hydrophilic monomer groups carrying hydrophobic groups, is between 0.1% and 10%, preferably between 0.5% and 5%, more preferably between 1% and 3%.

8. The process according to one of claims 1 to 7, **characterised in that** the hydrophobic groups comprise a carbon backbone comprising at least 8 carbon atoms.

9. The process according to claim 8, **characterised in that** the hydrophobic groups are hydrocarbon chains, in particular alkyls comprising 8 to 28 carbon atoms, preferably 12 to 18 carbon atoms.

10. The process according to one of claims 1 to 9, **characterised in that** the hydrophobic groups are bonded to the main chain by ether, ester or amide groups.

11. The process according to one of claims 1 to 10, **characterised in that** the concentration of the associative polymer is between 0.5% and 10% by weight when said polymer has a mass average molar mass of the order of 150000, this concentration being lower for a higher average molar mass.

12. The process according to one of claims 1 to 11, **characterised in that** said surfactant is a non ionic or ionic surfactant.

13. The process according to one of claims 1 to 12, **characterised in that** the surfactant is selected from the group constituted by polyethyleneglycol monoalkylethers of general formula:
CᵢH₂ᵢ₊₁(OCH₂CH₂)ⱼOH
where i is between 8 and 18, preferably equal to 12 or 14, and j is between 1 and 10, preferably between 3 and 5.

14. The process according to one of claims 1 to 12, **characterised in that** the surfactant is selected from the group constituted by polyglycerol monoalkylethers, in particular of formula:
R⁰O-[C₃H₅(OH)O-]ₙ-H
in which:
i) C₃H₅(OH)O is represented by one of the following structures, taken as a mixture or separately: 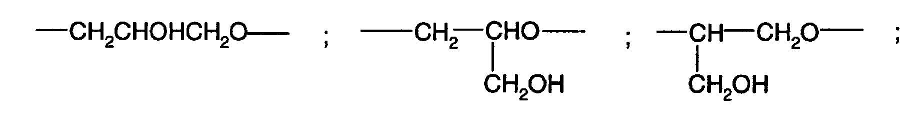
ii) n is an average statistical value between 1 and 6;
iii) R⁰ represents:
a) a linear or branched, saturated or unsaturated aliphatic chain containing 12 to 30 carbon atoms; or a hydrocarbon radical of a lanolin alcohol; or a long-chain α-diol residue;
b) an R¹CO residue, where R¹ is a linear or branched aliphatic C₁₁-C₁₇ radical;
c) an R²-[OC₂H₃(R³)-] residue, where:
· R² can have the meaning given in a) or b) for R⁰;
· OC₂H₃(R³)- is represented by one of the following structures, taken as a mixture or separately: 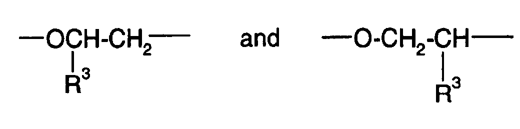
where R³ has the meaning given in a) for R⁰;
or it may be a linear or branched polyglycerol ether comprising two fatty chains.

15. The process according to one of claims 1 to 12, **characterised in that** the surfactant is an ionic surfactant such as a soap, for example sodium oleate, lecithin such as soya or egg lecithin, or a quaternary ammonium derivative such as a dialkyldimethylammonium type compound, the alkyl group being a fatty chain of at least 8 carbon atoms, for example dioctadecyldimethylammonium bromide.

16. The process according to one of claims 1 to 15, **characterised in that** the surfactant can form liposomes, vesicles or lamellar phases.

17. The process according to one of claims 1 to 16, **characterised in that** the composition further contains a compound intended for controlling bilayer formation, for example a second surfactant, a fatty alcohol, a sterol, a fatty alcohol or sterol phosphate, a fatty alcohol or sterol sulphate, a fatty acid, a fatty amine, a saponin, a triterpene derivative, a ceramide, or a sphingosine.

18. Use of the process according to one of claims 1 to 17 for stabilising an aqueous suspension of liquid or solid particles, by increasing its viscosity.

19. Use according to claim 18, **characterised in that** said particles are constituted by droplets of a liquid which are not miscible with water and have a dimension between 0.05 microns and 10 microns, and that it is for example an oil-in-water emulsion.

20. Use according to claim 18, **characterised in that** said particles are solid particles, in particular pigments or talcs, or micas, which may have been surface treated, with a dimension between 0.02 microns and 50 microns.

21. Use according to claim 18, **characterised in that** said suspension is a dispersion organised in the form of bilayers such as a lamellar phase, vesicles or liposomes.

22. Use according to any one of claims 18 to 21, **characterised in that** it is carried out for the preparation of a cosmetic composition.

23. A process for stabilising a suspension of vesicles by increasing its viscosity, especially a suspension of liposomes, **characterised in that** it consists in adding to said suspension an associative polymer as defined in one of the preceding claims.
